(19) Europäisches Patentamt
European Patent Office
Office européen des brevets

(11) Veröffentlichungsnummer: **0 020 954**
**A1**

(12) **EUROPÄISCHE PATENTANMELDUNG**

(21) Anmeldenummer: **80102449.8**

(22) Anmeldetag: **06.05.80**

(51) Int. Cl.³: **C 07 C 69/743**, C 07 C 43/17, C 07 C 61/35, A 01 N 53/00

(30) Priorität: **16.05.79 DE 2919820**

(43) Veröffentlichungstag der Anmeldung: **07.01.81 Patentblatt 81/1**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Fuchs, Rainer, Dr., Roeberstrasse 8, D-5600 Wuppertal 1 (DE)**
Erfinder: **Hammann, Ingeborg, Dr., Belfortstrasse 9, D-5000 Köln (DE)**
Erfinder: **Homeyer, Bernhard, Dr., Obere Strasse 28, D-5090 Leverkusen 3 (DE)**
Erfinder: **Behrenz, Wolfang, Dr., Untergründemich 14, D-5063 Overath (DE)**
Erfinder: **Stendel, Wilhelm, Dr., In den Birken 55, D-5600 Wuppertal 1 (DE)**

(54) **Fluor-substituierte Oxyalkenyl-cyclopropancarbonsäureester, Verfahren zu ihrer Herstellung, Zwischenverbindungen dafür, deren Herstellung, die Endprodukte enthaltende Insektizide und/oder Akarizide, deren Herstellung und Verwendung.**

(57) Gegenstand der vorliegenden Erfindung sind (1) neue fluor-substituierte Oxalkenyl-cyclopropancarbonsäureester der Formel (I)

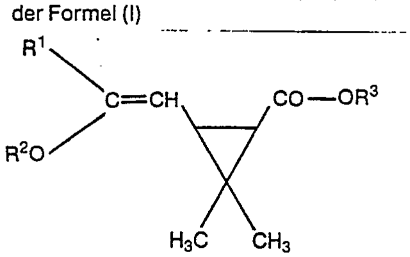

(I)

in welcher $R^1$, $R^2$ und $R^3$ die in der Beschreibung angegebene Bedeutung haben. Sie werden hergestellt, indem man (2) fluor-substituierte Oxalkenyl-cyclopropancarbonsäuren der Formel (II)

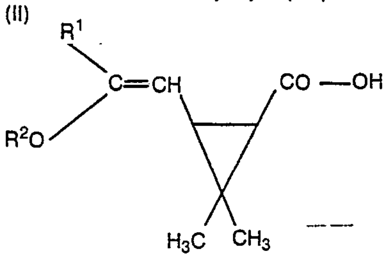

(II)

oder reaktionsfähige Derivate derselben mit Alkoholen der Formel (III)

$HO-R^3$ (III)

umsetzt. Die neuen fluor-substituierten Oxalkenyl-cyclopropan-carbonsäureester der Formel (I) zeichnen sich durch hohe insektizide und akarizide Wirksamkeit aus. Gegenstand der Erfindung sind ferner neue Zwischenprodukte zur Herstellung der Verbindungen der Formel (I).

EP 0 020 954 A1

BAYER AKTIENGESELLSCHAFT
Zentralbereich
Patente, Marken und Lizenzen

5090 Leverkusen, Bayerwerk
Rt/AB/Kü
Typ Ia

**BEZEICHNUNG GEÄNDERT**
**siehe Titelseite**

Fluor-substituierte Oxyalkenyl-cyclopropancarbonsäureester, Verfahren zu ihrer Herstellung sowie ihre Verwendung als Insektizide und Akarizide

Die Erfindung betrifft neue fluor-substituierte Oxyalkenyl-cyclopropancarbonsäureester, ein Verfahren zu ihrer Herstellung und Zwischenprodukte dafür sowie ihre Verwendung als Insektizide und Akarizide.

Es ist bekannt, daß bestimmte Alkenyl-cyclopropancarbonsäureester, wie z.B. 3-(2-Methyl-propen-1-yl)-2,2-dimethyl-cyclopropan-1-carbonsäure-(3-phenoxy-benzyl)-ester und 3-(2,2-Dichlor-vinyl)-2,2-dimethylcyclopropan-1-carbonsäure-(3-phenoxy-benzyl)-ester insektizid und akarizid wirksam sind (vergleiche DE-OS' 1 926 433 und 2 326 o77). Die Wirkung dieser Verbindungen ist jedoch, insbesondere bei niedrigen Wirkstoffkonzentrationen und Aufwandmengen, nicht immer zufriedenstellend.

Gegenstand der vorliegenden Erfindung sind

(1) neue fluor-substituierte Oxyalkenyl-cyclopropancarbonsäureester der Formel I



BAD ORIGINAL

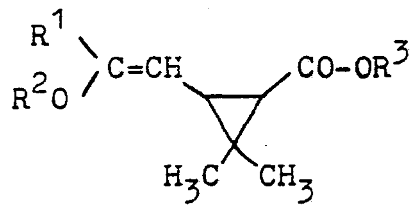

(I)

in welcher

$R^1$ für Alkyl steht, das durch mindestens ein Fluoratom substituiert ist,

$R^2$ für gegebenenfalls substituierte Reste Alkyl, Cycloalkyl, Aralkyl oder Aryl steht und

$R^3$ für einen in der Alkoholkomponente von Pyrethroiden üblichen Rest steht;

(2) ein Verfahren zur Herstellung von Verbindungen der Formel (I), dadurch gekennzeichnet, daß man fluor-substituierte Oxyalkenyl-cyclopropancarbonsäuren der Formel II

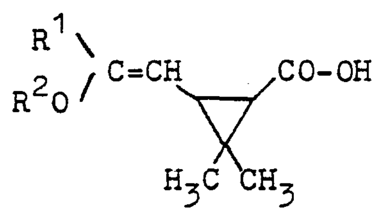

(II)

in welcher

$R^1$ und $R^2$ die oben angegebenen Bedeutungen haben,

oder reaktionsfähige Derivate derselben,

mit Alkoholen der Formel III

$$HO-R^3 \qquad (III)$$

in welcher

$R^3$ die oben angegebene Bedeutung hat,

oder mit reaktionsfähigen Derivaten derselben,

gegebenenfalls in Gegenwart eines Säureakzeptors und gegebenenfalls unter Verwendung eines oder mehrerer Verdünnungsmittel umsetzt;

(3) neue fluor-substituierte Oxyalkenyl-cyclopropancarbonsäuren der Formel II

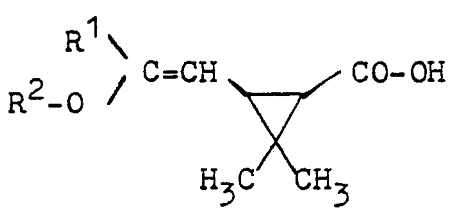

(II)

in welcher

$R^1$ und $R^2$ die unter (1) angegebenen Bedeutungen haben;

(4) ein Verfahren zur Herstellung von fluor-substituierten Oxyalkenyl-cyclopropancarbonsäuren der Formel (II), dadurch gekennzeichnet, daß man fluor-substituierte Oxy-diene der Formel IV

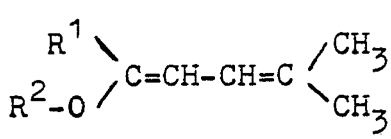

(IV)

in welcher

$R^1$ und $R^2$ die unter (1) angegebenen Bedeutungen haben,

mit Diazoessigsäureestern der Formel V

$$N_2CH-CO-OR^4 \qquad (V)$$

in welcher

$R^4$ für $C_1$-$C_4$-Alkyl steht,

in Gegenwart eines Katalysators umsetzt und die auf diese Weise hergestellten Ester der Formel II a

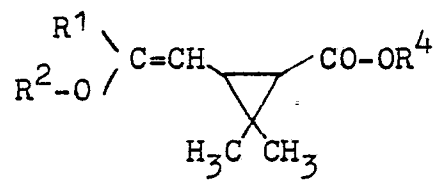

(II a)

in welcher

$R^1$, $R^2$ und $R^4$ die oben angegebenen Bedeutungen haben,

nach üblichen Methoden, durch Erhitzen mit wässrigalkoholischen Alkalilaugen, zu den entsprechenden Carbonsäuren der Formel (II) verseift;

(5) neue fluor-substituierte Oxy-diene der Formel IV

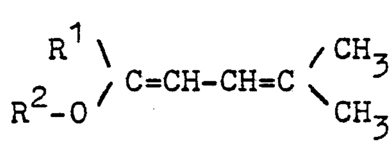

(IV)

in welcher

$R^1$ und $R^2$ die unter (1) angegebenen Bedeutungen haben,

(6) ein Verfahren zur Herstellung von fluor-substituierten Oxy-dienen der Formel (IV), dadurch gekennzeichnet, daß man fluor-substituierte Carbonsäureester der Formel VI

$$R^1\text{-CO-OR}^2 \qquad (VI)$$

in welcher

$R^1$ und $R^2$ die oben angegebenen Bedeutungen haben,

mit 3-Methyl-but-2-enyl-triphenyl-phosphoniumbromid der Formel VII

$$(C_6H_5)_3\overset{\oplus}{P}\text{-CH}_2\text{-CH=C}\begin{matrix}\diagup CH_3\\ \diagdown CH_3\end{matrix} \quad Br^{\ominus} \qquad (VII)$$

in Gegenwart einer starken Base und gegebenenfalls unter Verwendung von Verdünnungsmitteln umsetzt.

Die neuen fluor-substituierten Oxyalkenyl-cyclopropancarbonsäureester der Formel (I) zeichnen sich durch hohe insektizide und akarizide Wirksamkeit aus.

Überraschenderweise zeigen die erfindungsgemäßen Verbindungen der Formel (I) eine erheblich höhere insektizide und akarizide Wirkung als aus dem Stand der Technik bekannte Verbindungen analoger Konstitution und gleicher Wirkungsrichtung.

Gegenstand der Erfindung sind vorzugsweise Verbindungen der Formel (I), in welcher

$R^1$ für $C_1$-$C_2$-Fluoralkyl oder $C_1$-$C_2$-Chlor-fluor-alkyl steht,

$R^2$ für $C_1$-$C_4$-Alkyl, $C_1$-$C_4$-Halogenalkyl, $C_5$-$C_6$-Cycloalkyl oder Benzyl steht, und

$R^3$ für einen der nachstehenden, in der Alkoholkomponente von Pyrethroiden üblichen Rest steht,

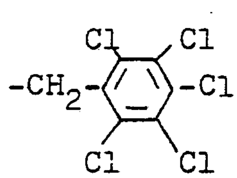 , 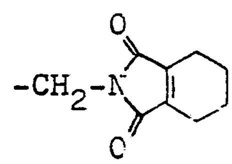 , 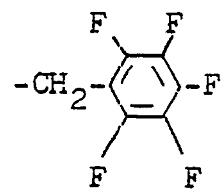

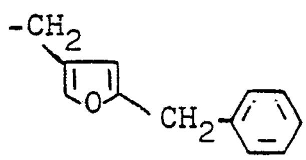 , 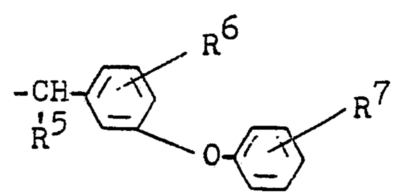 ;

wobei

$R^5$ für Wasserstoff, Cyano, Äthinyl, Methyl oder Äthyl steht und

$R^6$ und $R^7$, welche gleich oder verschieden sein können, für Wasserstoff oder Halogen stehen.

Die allgemeine Formel (I) schließt die verschiedenen möglichen Stereoisomeren und optisch aktiven Isomeren sowie deren Mischungen mit ein.

Die Herstellung der neuen Verbindungen der Formel (I) erfolgt bevorzugt in einer Variante a) des unter 2 (oben) angegebenen Verfahrens, indem als reaktionsfähige Derivate der Carbonsäuren der Formel (II) die entsprechenden Carbonsäurechloride der Formel II b

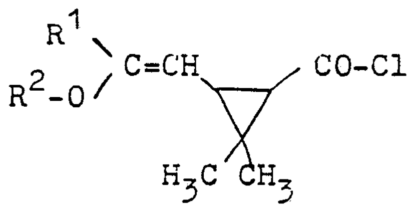

(II b)

in welcher

$R^1$ und $R^2$ die unter (1) angegebenen Bedeutungen haben,

mit Alkoholen der Formel III (oben), gegebenenfalls in Gegenwart eines Säureakzeptors und gegebenenfalls unter Verwendung eines Verdünnungsmittels umgesetzt werden.

Die Herstellung der neuen Verbindungen der Formel (I), in welcher $R^3$ für gegebenenfalls halogen-substituiertes 3-Phenoxy-$\alpha$-cyano-benzyl steht, erfolgt besonders bevorzugt, indem man b) Carbonsäurechloride der Formel II (oben) mit gegebenenfalls halogensubstituierten 3-Phenoxy-benzaldehyden der Formel VIII

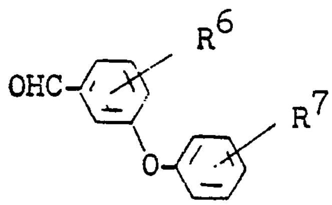

(VIII)

in welcher

$R^6$ und $R^7$ die oben angegebenen Bedeutungen haben,

und wenigstens der äquimolaren Menge Alkalicyanid (vorzugsweise Natriumcyanid oder Kaliumcyanid), gegebenenfalls in Gegenwart eines Katalysators und gegebenenfalls unter Verwendung von Verdünnungsmitteln umsetzt.

Verwendet man als Ausgangsstoffe bei der Verfahrensvariante (a) beispielsweise 3-(2-Methoxy-3,3,3-trifluor-propen-1-yl)-2,2-dimethyl-cyclopropan-1-carbonsäurechlorid und 3-Phenoxy-benzylalkohol, und bei Variante (b) 3-(2-Äthoxy-3,3,3-trifluor-propen-1-yl)-2,2-dimethyl-cyclopropan-1-carbonsäurechlorid, Natriumcyanid und 4-Fluor-3-phenoxy-benzaldehyd, so können die entsprechenden Reaktionen durch folgende Formelschemata skizziert werden:

(a)

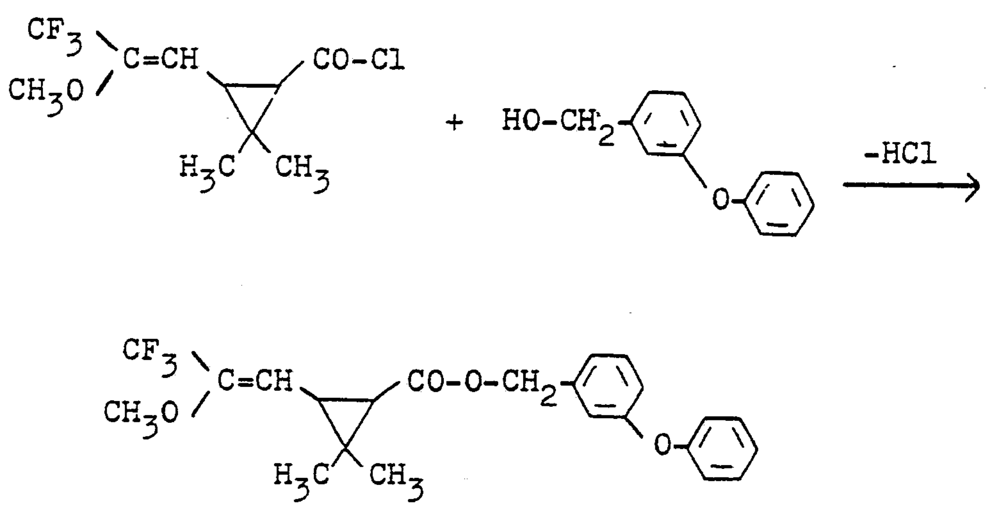

(b)

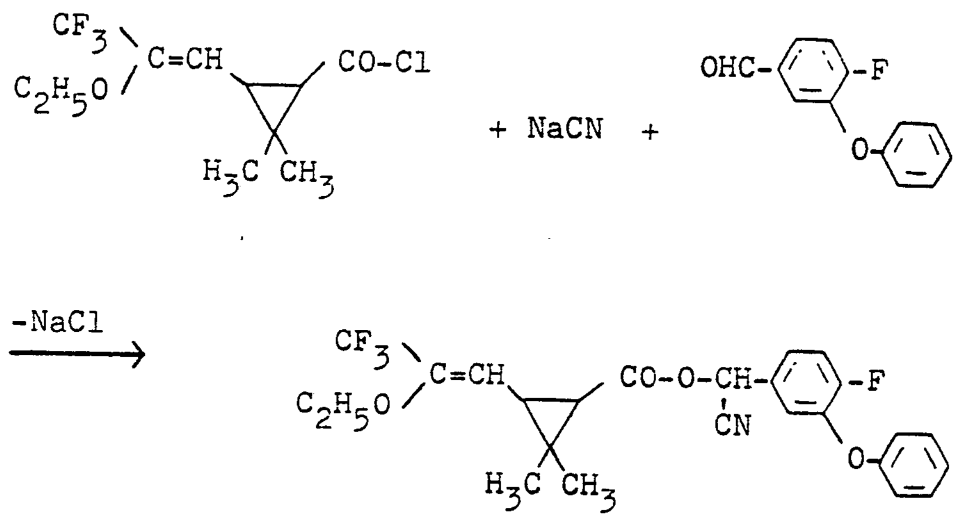


Das Verfahren zur Herstellung der neuen fluor-substituierten Oxyalkenyl-cyclopropancarbonsäureester (I) wird in allen Varianten bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Pentan, Hexan, Heptan, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Beim Arbeiten im Zweiphasenmedium wird Wasser als zweite Solvenskomponente verwendet.

Bei der Variante (a) des unter (2) beschriebenen Herstellungsverfahrens können als Säureakzeptoren alle üblichen Säurebindemittel Verwendung finden.

Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Bei der Variante (b) von Verfahren (2) werden als Katalysatoren Verbindungen verwendet, welche gewöhnlich bei Reaktionen in mehrphasigen Medien als Hilfsmittel zum Phasentransfer von Reaktanden dienen. Insbesondere seien Tetraalkyl- und Trialkylaralkyl-ammoniumsalze wie z.B. Tetrabutylammoniumbromid und Trimethyl-benzylammoniumchlorid genannt.

Die Reaktionstemperatur kann innerhalb eines größeren Bereichs variiert werden. Im allgemeinen arbeitet man zwischen 0 und 1oo°C, bei Verfahrensvariante (a) vorzugsweise zwischen lo und 5o°C, bei Variante (b) vorzugsweise zwischen lo und 3o°C.

Alle Varianten des erfindungsgemäßen Verfahrens werden im allgemeinen bei Normaldruck durchgeführt.

Zur Durchführung des erfindungsgemäßen Verfahrens (2) werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem oder mehreren Verdünnungsmitteln in Gegenwart eines Säureakzeptors oder eines Katalysators durchgeführt und das Reaktionsgemisch wird mehrere Stunden bei der erforderlichen Temperatur gerührt. Anschließend wird das Reaktionsgemisch mit Toluol/Wasser geschüttelt, die organische Phase abgetrennt, mit Wasser gewaschen und getrocknet.

Nach Abdestillieren des Lösungsmittels im Vakuum fallen im allgemeinen die neuen Verbindungen in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die Ausgangsstoffe für die Herstellung der erfindungsgemäßen Verbindungen sind durch die Formeln (II), (II a), (II b), (III), (IV), (V), (VI), (VII) und (VIII) definiert. Vorzugsweise stehen darin die Reste $R^1$ bis $R^3$ und $R^5$ bis $R^7$ für diejenigen Reste, welche bei der Definition der Reste $R^1$ bis $R^3$ und $R^5$ bis $R^7$ in Formel (I) als bevorzugt genannt wurden, und $R^4$ steht für $C_1$-$C_4$-Alkyl.

Alkohole der Formel (III) bzw. Aldehyde der Formel (VIII) als davon abzuleitende Verbindungen, welche bei dem oben unter (2) angegebenen Verfahren zur Herstellung der neuen fluor-substituierten Oxyalkenyl-cyclopropan-carbonsäureester der Formel (I) als Reaktionskomponenten einzusetzen sind, sind bekannt (vergleiche DE-OS' 2 oo5 489, 2 326 o77, 2 7o9 264; GB-PS 1 o78 511).

Als Beispiele seien genannt:
Tetrahydrophthalimidomethylalkohol, Pentafluorbenzylalkohol, Pentachlorbenzylalkohol, 5-Benzyl-3-furylalkohol, 3-Phenoxy-benzylalkohol, 3-Phenoxy-benzaldehyd, 3-(3-Fluoro-phenoxy)-, 3-(4-Fluoro-phenoxy)-, 3-Phenoxy-4-fluoro-, 3-(3-Fluoro-phenoxy)-4-fluoro- und 3-(4-Fluoro-phenoxy)-4-fluoro-benzylalkohol,
3-(3-Fluoro-phenoxy)-, 3-(4-Fluoro-phenoxy)-, 3-Phenoxy-4-fluoro-, 3-(3-Fluoro-phenoxy)-4-fluoro- und 3-(4-Fluoro-phenoxy)-4-fluoro-α-cyano-benzylalkohol,
3-(3-Fluoro-phenoxy)-, 3-(4-Fluoro-phenoxy)-, 3-Phenoxy-4-fluoro-, 3-(3-Fluoro-phenoxy)-4-fluoro- und 3-(4-Fluoro-phenoxy)-4-fluoro-α-äthinyl-benzylalkohol,
3-Phenoxy-4-fluoro-α-methyl-benzylalkohol sowie
3-(3-Fluoro-phenoxy)-, 3-(4-Fluoro-phenoxy)-, 3-Phenoxy-4-fluoro-, 3-(3-Fluoro-phenoxy)-4-fluoro- und 3-(4-Fluoro-phenoxy)-4-fluoro-benzaldehyd.

Die als Ausgangsverbindungen zu verwendenden fluorsubstituierten Oxyalkenyl-cyclopropancarbonsäuren (II) bzw. die entsprechenden Ester (IIa) bzw. die entsprechenden Säurechloride (IIb) als deren reaktionsfähige Derivate sind neu.

Carbonsäurechloride der Formel (II b) können nach üblichen Methoden aus den entsprechenden Carbonsäuren der Formel (II), durch Umsetzung mit einem Chlorierungsmittel wie z.B. Thionylchlorid, gegebenenfalls unter Verwendung eines Verdünnungsmittels wie z.B. Tetrachlorkohlenstoff, bei Temperaturen zwischen lo und loo°C hergestellt werden.

Die neuen Carbonsäuren der Formel (II) enthält man aus den entsprechenden Alkylestern der Formel (II a) durch mehrstündiges Erhitzen mit wässrig-alkoholischer Alkalilauge wie z.B. Natronlauge, auf Temperaturen zwischen 5o und 15o°C. Die Aufarbeitung erfolgt nach üblichen Methoden, beispielsweise durch Abdestillieren des Alkohols, Verdünnen mit Wasser, Ansäuern, Extrahieren mit Methylenchlorid, Trocknen der organischen Extrakte und Abdestillieren des Lösungsmittels.

Die neuen Fluor-substituierten Oxyalkenyl-cyclopropancarbonsäureester der Formel (II a) erhält man, wie oben unter (4) erläutert, durch Umsetzung von fluor-substituierten Oxy-dienen der Formel IV (oben) mit Diazoessigsäureestern der Formel V (oben) in Gegenwart eines Katalysators bei Temperaturen zwischen 5o und 2oo°C, vorzugsweise zwischen 8o und 15o°C. Als Katalysatoren werden vorzugsweise Kupfer, Kupferverbindungen oder Gemische aus Kupfer und Kupferverbindungen, wie z.B. Kupferpulver und Kupfersulfat verwendet.

Zur Aufarbeitung wird das Reaktionsgemisch mit Methylenchlorid verdünnt und filtriert. Vom Filtrat wird nach Waschen mit Wasser und Trocknen das Lösungsmittel abgezogen und das zurückbleibende Rohprodukt gegebenenfalls durch Destillation unter vermindertem Druck gereinigt.

Als Beispiele für die Carbonsäuren der Formel (II), die entsprechenden Ester (II a) und Säurechloride (II b) seien genannt:

3-(2-Methoxy-3,3,3-trifluor-propen-1-yl)-,
3-(2-Äthoxy-3,3,3-trifluor-propen-1-yl)-,
3-(2-n-Propoxy-3,3,3-trifluor-propen-1-yl)-,
3-(2-iso-Propoxy-3,3,3-trifluor-propen-1-yl)- und
3-(2-(2-Chlor-äthoxy)-3,3,3-trifluor-propen-1-yl)-
-2,2-dimethyl-cyclopropan-1-carbonsäure sowie die entsprechenden Säurechloride, die Methylester und die Äthylester.

Die oben unter (5) definierten neuen fluor-substituierten Oxy-diene der Formel (IV) erhält man, wie oben unter (6) angegeben, durch Umsetzung von fluor-substituierten Carbonsäureestern der Formel VI (oben) mit 3-Methyl-but-2-enyl-triphenyl-phosphoniumbromid in

Gegenwart einer starken Base wie z.B. Butyllithium, unter Verwendung von Verdünnungsmitteln wie z.B. Tetrahydrofuran und Hexan, bei Temperaturen zwischen -7o und +1oo°C, vorzugsweise zwischen -2o und +5o°C.

Zur Aufarbeitung wird das Reaktionsgemisch mit Wasser verdünnt und mit einem mit Wasser nicht mischbaren Lösungsmittel wie z.B. Petroläther mehrfach extrahiert. Von den vereinigten Extrakten wird nach Trocknen das Lösungsmittel abgezogen. Das im Rückstand verbleibende Rohprodukt wird gegebenenfalls durch Vakuumdestillation gereinigt.

Als Beispiele für die fluor-substituierten Oxy-diene der Formel (IV) seien genannt:
1,1,1-Trifluor-2-methoxy-5-methyl-hexa-2,4-dien,
1,1,1-Trifluor-2-äthoxy-5-methyl-hexa-2,4-dien,
1,1,1-Trifluor-2-n-propoxy-5-methyl-hexa-2,4-dien,
1,1,1-Trifluor-2-iso-propoxy-5-methyl-hexa-2,4-dien und
1,1,1-Trifluor-2-(2-chlor-äthoxy)-5-methyl-hexa-2,4-dien.

Fluor-substituierte Carbonsäureester der Formel (VI ), welche als Ausgangsstoffe für das unter (6) angegebene Verfahren zu verwenden sind, sind bekannt (vergleiche J.Am.Chem.Soc. 69 (1947), 1819).

Als Beispiele seien Trifluoressigsäure-methylester, -äthylester, -n-propylester und -iso-propylester genannt.

3-Methyl-but-2-enyl-triphenylphosphoniumbromid (VII) ist ebenfalls bekannt (vergleiche Helv. Chim. Acta 44 (1961), 998-9).

Die neuen fluor-substituierten Oxyalkenyl-cyclopropan-carbonsäureester (I) zeichnen sich, wie bereits erwähnt, durch hohe insektizide und akarizide Wirksamkeit aus. Sie können gegen pflanzenschädigende Insekten und Milben in Land- und Forstwirtschaft, im Vorratsschutz und Hygienebereich sowie gegen Ektoparasiten im veterinär-medizinischen Bereich eingesetzt werden.

Die Wirkstoffe eignen sich bei guter Pflanzenverträglichkeit und günstiger Warmblütertoxizität zur Bekämpfung von tierischen Schädlingen, insbesondere Insekten und Spinnentieren, die in der Landwirtschaft, in Forsten, im Vorrats- und Materialschutz sowie auf dem Hygienesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix,

Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.
Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.
Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.
Aus der Ordnung der Coleoptera z.B. Anobium punctatum, Rhizopertha dominica, Bruchidius obtectus, Acanthoscelides

obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die erfindungsgemäßen Wirkstoffe eignen sich auch zur Bekämpfung von Ekto- und Endoparasiten auf dem veterinärmedizinischen Gebiet.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuders sowie durch parenterale Anwendung in Form beispielsweise der Injektion.

Beispiel A

Plutella-Test

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1,3.

Beispiel B

Myzus-Test

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Blattläuse abgetötet wurden; 0 % bedeutet, daß keine Blattläuse abgetötet wurden.

Bei diesem Test zeigen z.B. die folgenden Verbindungen der Herstellungsbeispiele überlegene Wirksamkeit gegenüber dem Stand der Technik: 17, 18.

Beispiel C

Tetranychus-Test (resistent)

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 17, 18, 3.

Beispiel D

Grenzkonzentrations-Test / Bodeninsekten

| | |
|---|---|
| Testinsekt: | Tenebrio molitor-Larven (im Boden) |
| Lösungsmittel: | 3 Gewichtsteile Aceton |
| Emulgator: | 1 Gewichtsteil Alkylarylpolyglykoläther |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit dem Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (= mg/l) angegeben wird. Man füllt den Boden in Töpfe und läßt diese bei Raumtemperatur stehen.

Nach 24 Stunden werden die Testtiere in den behandelten Boden gegeben und nach weiteren 2 bis 7 Tagen wird der Wirkungsgrad des Wirkstoffs durch Auszählen der toten und lebenden Testinsekten in % bestimmt. Der Wirkungsgrad ist 100 %, wenn alle Testinsekten abgetötet worden sind, er ist 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 18, 17, 3 .

Beispiel E

$LT_{100}$-Test für Dipteren

Testtiere: Aedes aegypti
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 17, 18

Beispiel F

$LT_{100}$-Test für Dipteren

Testtiere: Musca domestica, resistent
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 17, 18

Beispiel G

$LD_{100}$-Test

Testtiere: Sitophilus granarius
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird 3 Tage nach Ansetzen der Versuche kontrolliert. Bestimmt wird die Abtötung in %. Dabei bedeutet 100 %, daß alle Testtiere abgetötet wurden; 0 % bedeutet, daß keine Testtiere abgetötet wurden.

Bei diesem Test zeigen z.B. die folgenden Verbindungen der Herstellungsbeispiele überlegene Wirkung gegenüber dem Stand der Technik: 17,18

Beispiel

Test mit Boophilus microplus resistent

Lösungsmittel: 35 Gewichtsteile Äthylenglykolmonomethyläther
35 Gewichtsteile Nonylphenolpolylgkyoläther

Zur Herstellung einer zweckmäßigen Wirkstoffzubereitung vermischt man drei Gewichtsteile Wirkstoff mit sieben Gewichtsteilen des oben angegebenen Lösungsmittel-Gemisches und verdünnt das so erhaltene Konzentrat mit Wasser auf die gewünschte Konzentration.

10 adulte Boophilus microplus res. werden in die zu testende Wirkstoffzubereitung 1 Minute getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 1,3,17,18

Herstellungsbeispiele

Beispiel 1:

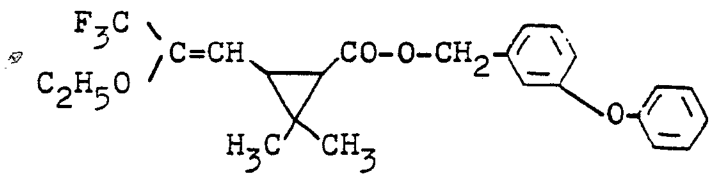

5,2 g (o,o26 Mol) 3-Phenoxy-benzylalkohol und 7 g (o,o26 Mol) 2,2-Dimethyl-3-(3,3,3-trifluor—2-äthoxy-propen-1-yl)-cyclopropancarbonsäurechlorid werden in 1oo ml wasserfreiem Toluol gelöst und bei 2o-25°C 2,5 g Pyridin, gelöst in 2o ml wasserfreiem Toluol, unter Rühren zugetropft. Anschließend wird weitere 3 Stunden bei 25-35°C gerührt. Das Reaktionsgemisch wird in 15o ml Wasser, dem 1o ml konzentrierte Salzsäure zugesetzt werden, gegossen, die organische Phase abgetrennt und nochmals mit 1oo ml Wasser gewaschen. Anschließend wird die Toluolphase über Natriumsulfat getrocknet und das Lösungsmittel im Wasserstrahlvakuum abdestilliert. Letzte Lösungsmittelreste werden durch kurzes Andestillieren bei 6o°C/1 Torr Badtemperatur entfernt. Man erhält 9,9 g (87,7% der Theorie) 2,2-Dimethyl-3-(3,3,3-trifluor--2-äthoxy-propen-1-yl)-cyclopropancarbonsäure-(3-phenoxy-benzyl)-ester als gelbes Öl mit dem Brechungsindex $n_D^{25}$: 1,513o.

Beispiel 2:

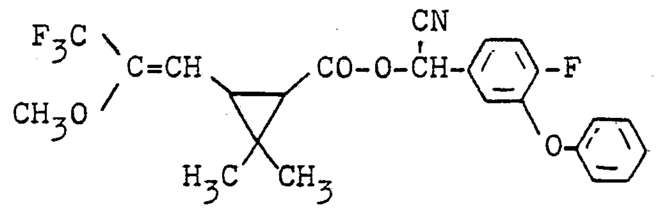

4,63 g (o,o214 Mol) 3-Phenoxy-4-fluor-benzaldehyd und 5,5 g (o,o214 Mol) 2,2-Dimethyl-3-(3,3,3-trifluor-2-methoxy-propen-1-yl)-cyclopropancarbonsäurechlorid werden zusammen unter Rühren bei 2o-25°C zu einer Mischung von 1,7 g Natriumcyanid, 2,5 ml Wasser, 1oo ml n-Hexan und o,5 g Tetrabutylammoniumbromid getropft und dann 4 Stunden bei 2o-25°C gerührt. Anschließend wird die Reaktionsmischung mit 3oo ml Toluol versetzt und 2 mal mit je 3oo ml Wasser ausgeschüttelt. Die organische Phase wird abgetrennt, über Magnesiumsulfat getrocknet und das Lösungsmittel im Wasserstrahlvakuum abdestilliert. Letzte Lösungsmittelreste werden durch kurzes Andestillieren bei 6o°C/1 Torr Badtemperatur entfernt. Man erhält 7,4 g (74,7% der Theorie) 2,2-Dimethyl-3-(3,3,3-trifluor-methyl-2-methoxy-propen-1-yl)-cyclopropancarbonsäure-(3-phenoxy-4-fluor-α-cyano-benzyl)-ester als zähes Öl mit dem Brechungsindex $n_D^{22}$: 1,5253.

Analog einem der vorstehenden Beispiele 1 oder 2 können folgende Verbindungen dargestellt werden:

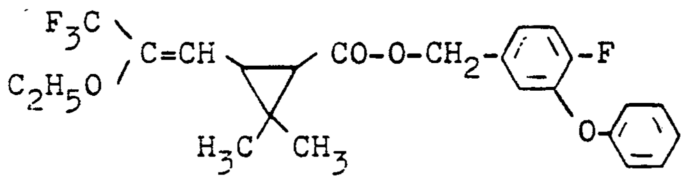

(3)

Brechungs-
index:
$n_D^{25}$ = 1,5085

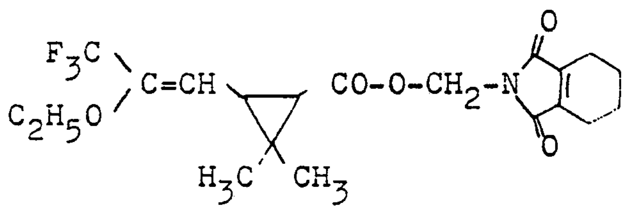

(4)

Brechungs-
index:
$n_D^{25}$ = 1,4840

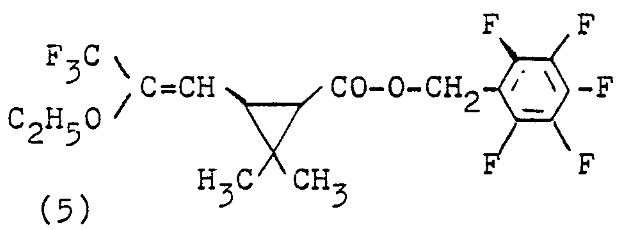

(5)

Brechungs-
index:
$n_D^{25}$ = 1,4439

Siedepunkt:
140-145°C/2 Torr

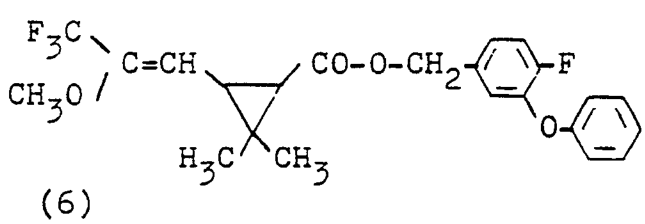

(6)

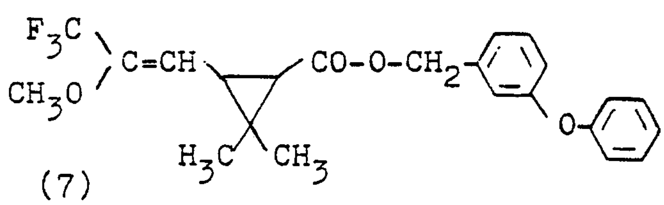

(7)

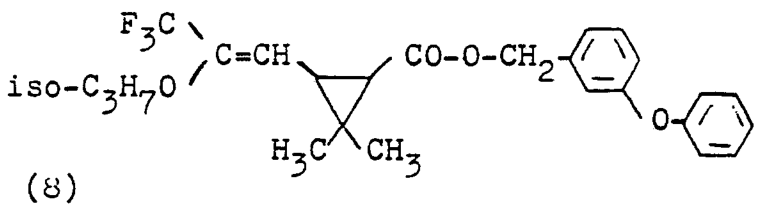

(8)

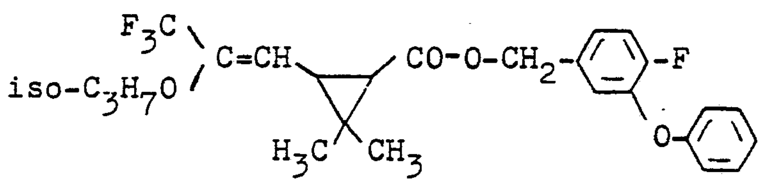

(9)

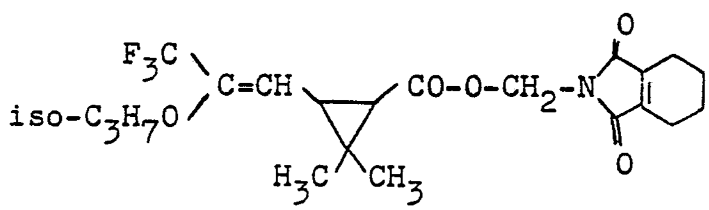

(1o)

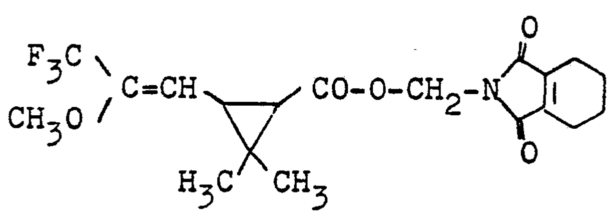

(11)

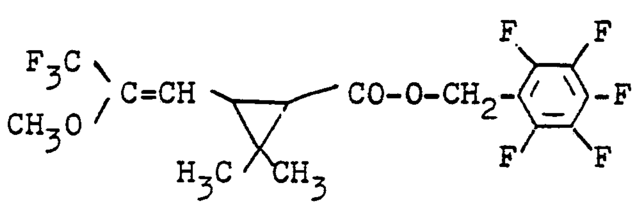

Brechungsindex
$n_D^{25} = 1,4450$

(12)

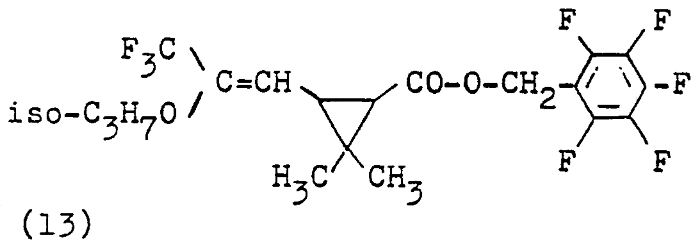

(13)

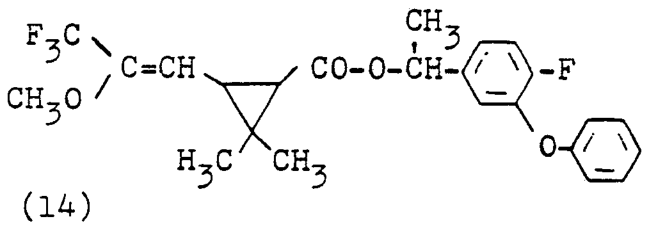

(14)

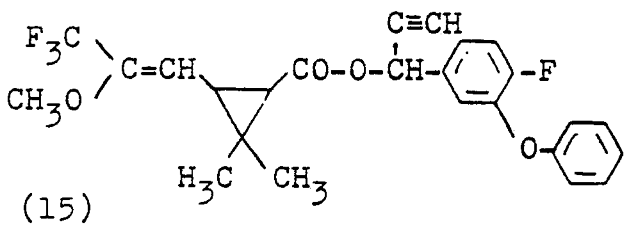

(15)

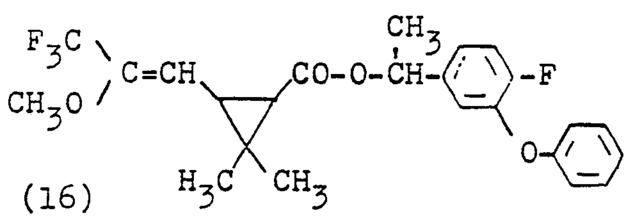

(16)

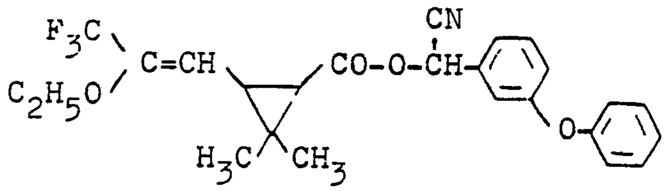

(17)

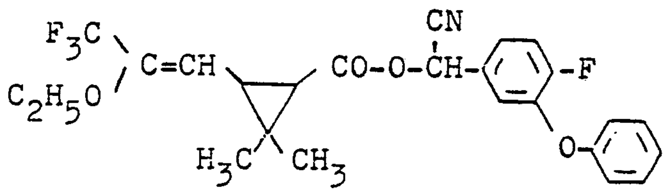

(18)

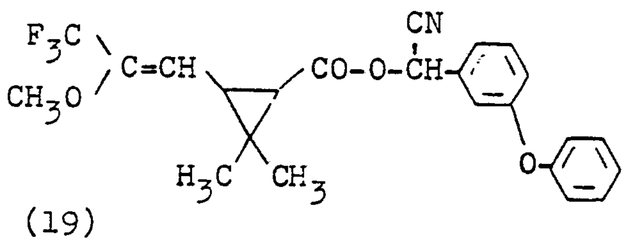

(19)

Brechungsindex
$n_D^{25}$ = 1,5230

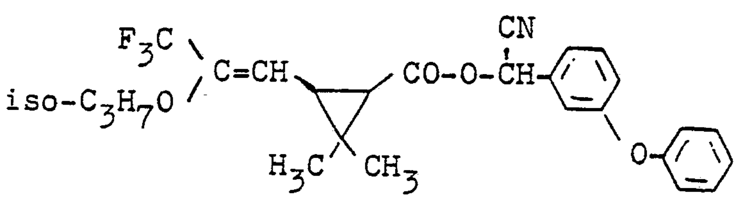

(2o)

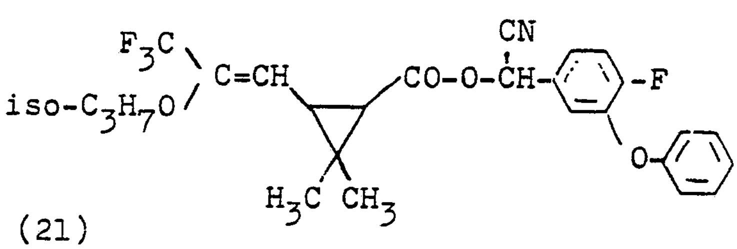

(21)

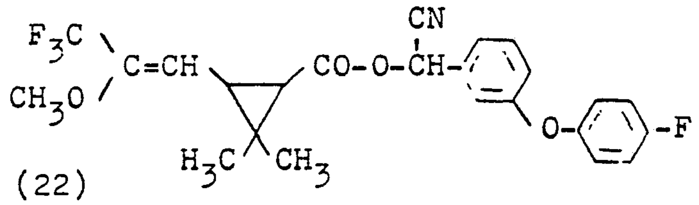

(22)

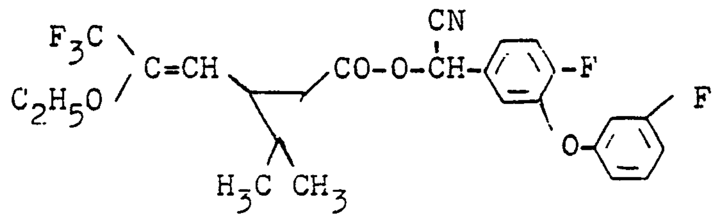

(23)

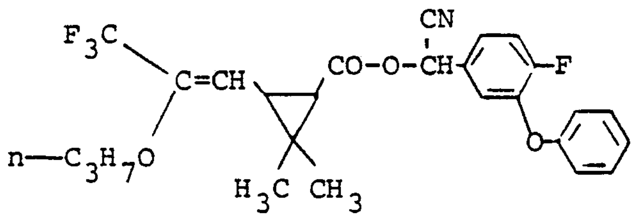

Brechungsindex:
$n_D^{25}$: 1,5130

(24)

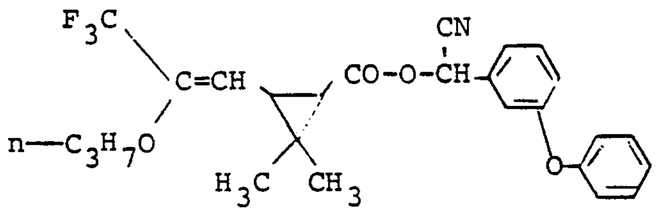

Brechungsindex:
$n_D^{25}$: 1,5195

(25)

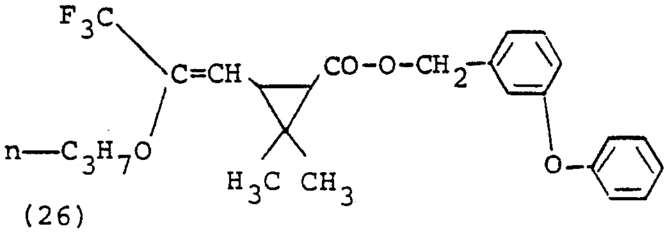

Brechungsindex:
$n_D^{25}$: 1,5070

(26)

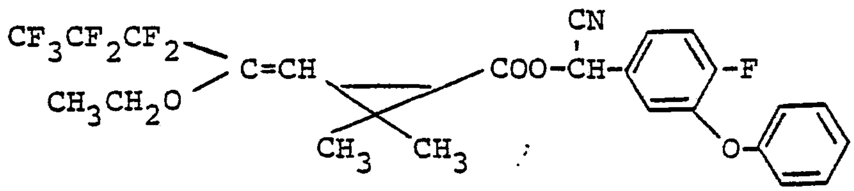

(27)

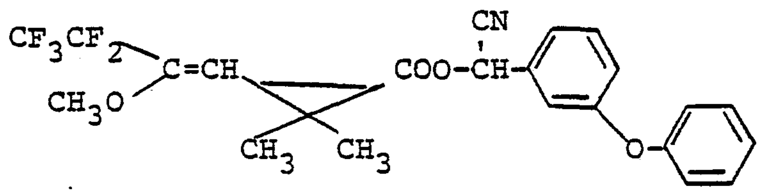

(28)

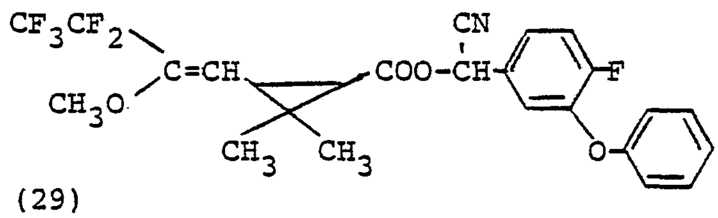

(29)

Die Ausgangsverbindungen können wie folgt hergestellt werden:

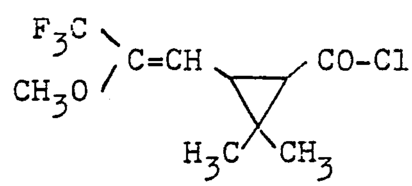

8,7 g (o,o365 Mol) 2,2-Dimethyl-3-(3,3,3-trifluor--2-methoxy-propen-1-yl)-cyclopropancarbonsäure werden in loo ml Tetrachlorkohlenstoff gelöst und bei 6o°C langsam unter Rühren 21 g Thionylchlorid zugetropft. Anschließend wird 4 Stunden zum Rückfluß erhitzt. Nach

beendeter Reaktionszeit werden überschüssiges Thionylchlorid sowie Tetrachlorkohlenstoff im Wasserstrahlvakuum abdestilliert. Der Rückstand wird im Vakuum destilliert. Man erhält 6 g (64,2% der Theorie) 2,2-Dimethyl-3-(3,3,3-trifluor--2-methoxy-propen-1-yl)-cyclopropancarbonsäurechlorid als leicht gelbe Flüssigkeit mit dem Siedepunkt 80°C/2 mBar.

Analog erhält man:

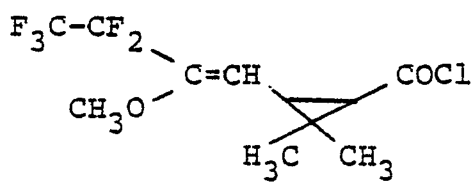

Siedepunkt: 92-93°C/2 mbar

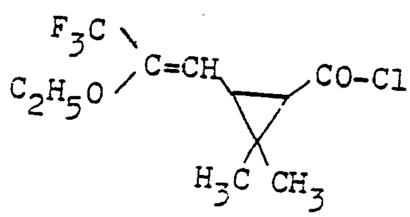

Siedepunkt: 90-91°C/2 mbar

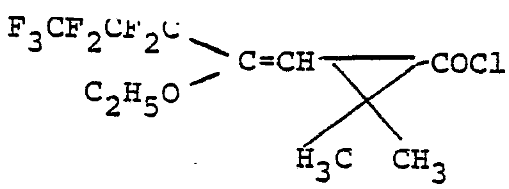

Siedepunkt: 115°C/ 6 mbar

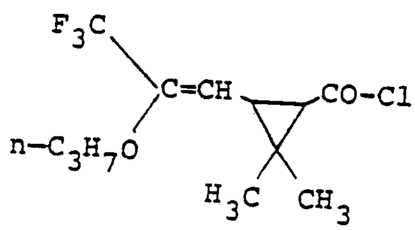

Siedepunkt: 91°C/2 mbar

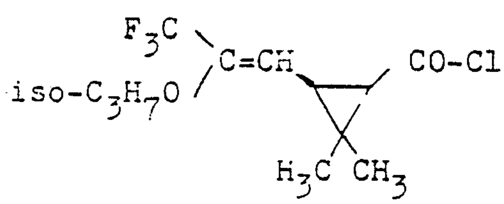

Siedepunkt: 93-94°C/2 mbar

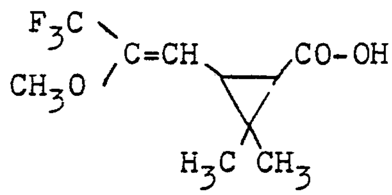

16,6 g (o,o624 Mol) 2,2-Dimethyl-3-(3,3,3-trifluor-2-methoxy-propen-1-yl)-cyclopropancarbonsäureäthylester werden in 1oo ml Äthanol gelöst, dann mit einer Lösung von 2,9 g (o,o73 Mol) Natriumhydroxid in 75 ml Wasser versetzt und 4 Stunden unter Rühren zum Rückfluß erhitzt. Anschließend wird das Äthanol im Wasserstrahlvakuum abdestilliert, der Rückstand in 3oo ml Wasser aufgenommen und 1 mal mit 3oo ml Methylenchlorid extrahiert. Die wässrige Phase wird abgetrennt, mit konzentrierter Salzsäure angesäuert und anschließend mit 2 mal 3oo ml Methylenchlorid extrahiert. Die organische Phase wird dann abgetrennt über Magnesiumsulfat, getrocknet und das Lösungsmittel im Wasserstrahlvakuum abdestilliert. Letzte Lösungsmittelreste werden durch kurzes Andestillieren bei 2 mBar/5o°C Badtemperatur entfernt. Man erhält dann 9 g (6o,6% der Theorie) 2,2-Dimethyl-3-(3,3,3-trifluor-2-methoxy-propen-1-yl)-cyclopropancarbonsäure als gelbes zähes Öl.

Analog erhält man:

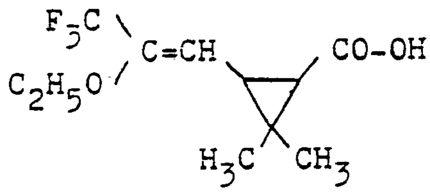

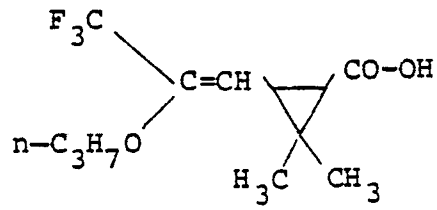

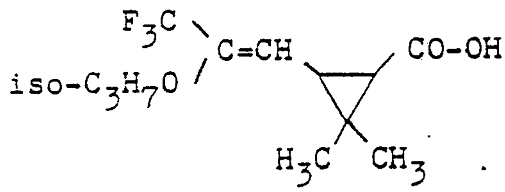

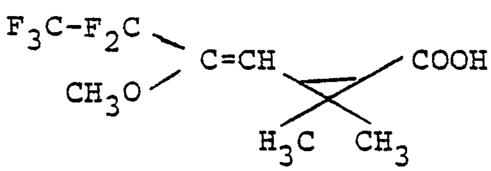

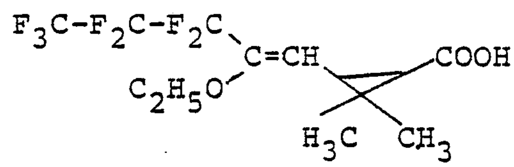

Beispiel:

Eine Mischung von 36 g (0,2 Mol) 1,1,1-Trifluor-2-methoxy-5-methyl-2,4-hexadien, 1,2 g Kupferpulver und 1,5 g Kupfersulfat (wasserfrei) wird auf 11o-12o°C erhitzt und dann unter Rühren ebenfalls bei 11o-12o°C eine Mischung von 18 g (o,1 Mol) 1,1,1-Trifluor—2-methoxy-5-methyl-2,4-hexadien und 34,2 g (o,3 Mol) Diazoessigsäureäthylester, sehr langsam, innerhalb von 6 Stunden, zugetropft. Nach beendeter Stickstoffentwicklung wird die Mischung abgekühlt, mit 5oo ml Methylenchlorid verdünnt und dann filtriert. Das Filtrat wird mit 5oo ml Wasser ausgeschüttelt, anschließend wird die organische Phase abgetrennt, über Magnesiumsulfat getrocknet und dann das Lösungsmittel im Wasserstrahlvakuum abdestilliert. Der ölige Rückstand wird im Vakuum destilliert. Man erhält dabei zwei Fraktionen:

Fraktion 1: Siedepunkt 62-65°C/36 mBar
Fraktion 2: Siedepunkt 5o-85°C/ 2 mBar

Fraktion 1 (16,6 g) erweist sich als nicht umgesetztes 1,1,1-Trifluor—2-methoxy-5-methyl-2,4-hexadien. Fraktion 2 wird nochmals destilliert. Man erhält 17,2 g (21,6% der Theorie) 2,2-Dimethyl-3-(3,3,3-trifluor—2-methoxy-propen-1-yl)-cyclopropancarbonsäureäthylester (Isomerengemisch) als farblose Flüssigkeit mit dem Siedepunkt: 8o-82°C/ 2 mBar.

Analog erhält man:

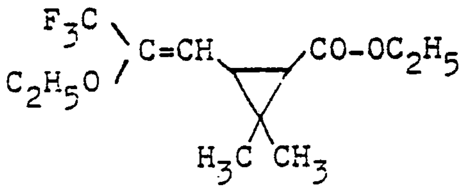

boiling point:
90-97°C/2 mbar

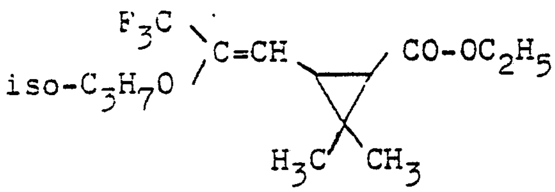

boiling point:
90-92°C/1 mbar

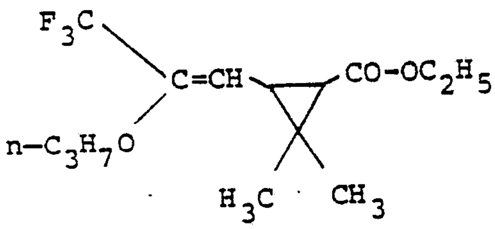

boiling point:
90-95°C/2 mbar

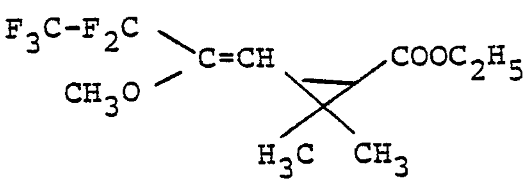

boiling point
90-95°C/2mbar

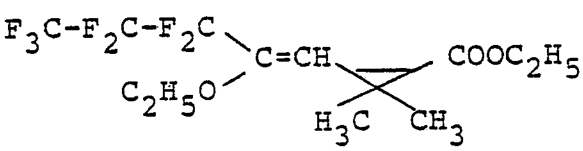

boiling point
110-116°C/2mbar

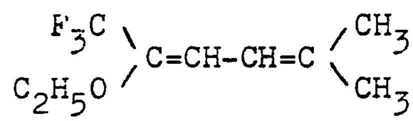

Zu einer Suspension von 82,2 g (o,2 Mol) trockenem 3,3-Dimethylallyl-triphenylphosphoniumbromid in 3oo ml wasserfreiem Tetrahydrofuran werden unter Stickstoff bei 0°C und unter Rühren 96 ml einer 2o%igen Lösung von n-Butyllithium in Hexan zugetropft. Die so erhaltene tiefrote Lösung wird noch 15 Minuten bei o°C gerührt und dann bei 0-1o°C 28,4 g (o,2 Mol) Trifluoressigsäureäthylester zugetropft. Anschließend wird solange bei Raumtemperatur gerührt, bis nahezu Entfärbung eingetreten ist (ca. 12 Stunden). Das Reaktionsgemisch wird dann mit 6oo ml Wasser versetzt und 5 mal mit je 2oo ml Petroläther extrahiert. Die Petrolätherphasen werden über Magnesiumsulfat getrocknet und dann das Lösungsmittel am Rotationsverdampfer im Wasserstrahlvakuum abgezogen. Der Rückstand wird mit 15o ml n-Hexan versetzt und dann filtriert. Anschließend wird vom Filtrat bei Normaldruck das Lösungsmittel abdestilliert und der ölige Rückstand dann im Vakuum destilliert. Man erhält 16,2 g (41,8 % der Theorie) 1,1,1-Trifluor-2-äthoxy-5-methyl-2,4-hexadien als leicht gelbliche Flüssigkeit mit dem Siedepunkt 72°C/ 36 mBar.

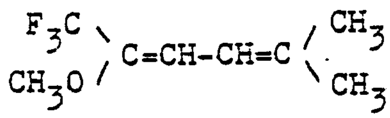

Schmelzpunkt
62°C/36 mbar

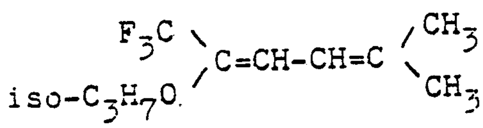

Schmelzpunkt
83°C/36 mbar

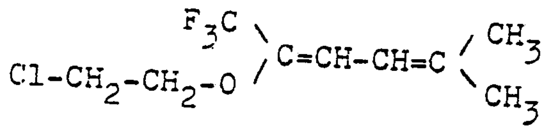

Schmelzpunkt
62-63°C/4 mbar

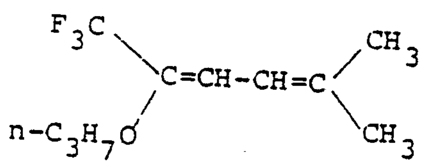

Schmelzpunkt
92-93°C/36 mbar

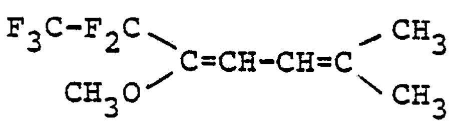

Schmelzpunkt
73-74°C/36 mbar

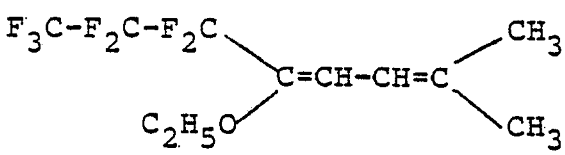

Schmelzpunkt
86-87°C/36 mbar

Patentansprüche

1. Fluor-substituierte Oxyalkenyl-cyclopropan-carbonsäureester der Formel I

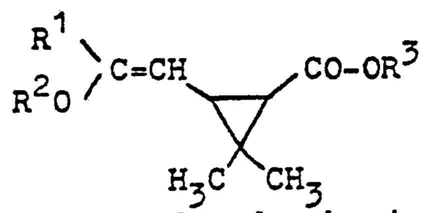

(I)

in welcher

$R^1$ für Alkyl steht, das durch mindestens ein Fluoratom substituiert ist,

$R^2$ für gegebenenfalls substituierte Reste Alkyl, Cycloalkyl, Aralkyl oder Aryl steht und

$R^3$ für einen in der Alkoholkomponente von Pyrethroiden üblichen Rest steht;

2. Verfahren zur Herstellung von Verbindungen der Formel (I), dadurch gekennzeichnet, daß man fluor-substituierte Oxyalkenyl-cyclopropancarbonsäuren der Formel II

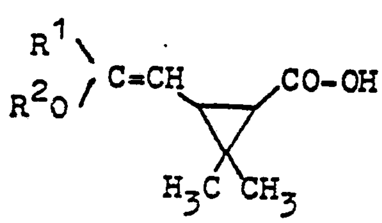

(II)

in welcher

$R^1$ und $R^2$ die oben angegebenen Bedeutungen haben,

oder reaktionsfähige Derivate derselben, mit Alkoholen der Formel III

$$HO\text{-}R^3 \qquad \text{(III)}$$

in welcher

$R^3$ die oben angegebene Bedeutung hat,

oder mit reaktionsfähigen Derivaten derselben, gegebenenfalls in Gegenwart eines Säureakzeptors und gegebenenfalls unter Verwendung eines oder mehrerer Verdünnungsmittel umsetzt.

3. Fluor-substituierte Oxyalkenyl-cyclopropan-carbonsäuren der Formel II

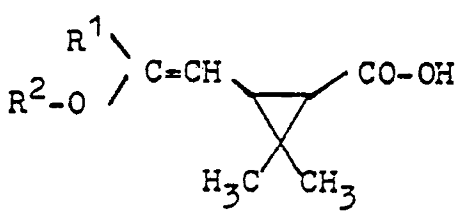

(II)

in welcher
$R^1$ und $R^2$ die unter (1) angegebenen Bedeutungen haben.

4. Verfahren zur Herstellung von fluor-substituierten Oxyalkenyl-cyclopropancarbonsäuren der Formel (II), dadurch gekennzeichnet, daß man fluor-substituierte Oxy-diene der Formel IV

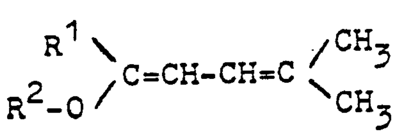

(IV)

in welcher

$R^1$ und $R^2$ die unter (1) angegebenen Bedeutungen haben,

mit Diazoessigsäureestern der Formel V

$$N_2CH\text{-}CO\text{-}OR^4 \qquad (V)$$

in welcher

$R^4$ für $C_1$-$C_4$-Alkyl steht,

in Gegenwart eines Katalysators umsetzt und die auf diese Weise hergestellten Ester der Formel II a

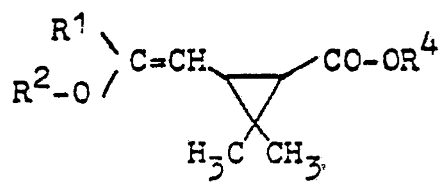

(II a)

in welcher

$R^1$, $R^2$ und $R^4$ die oben angegebenen Bedeutungen haben,

nach üblichen Methoden, durch Erhitzen mit wässrigalkoholischen Alkalilaugen, zu den entsprechenden Carbonsäuren der Formel (II) verseift

5. Fluor-substituierte Oxy-diene der Formel IV

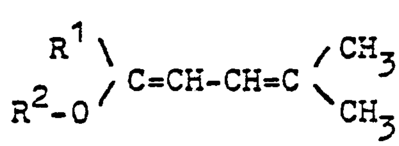

(IV)

in welcher

$R^1$ und $R^2$ die unter (1) angegebenen Bedeutungen haben,

6. Verfahren zur Herstellung von fluor-substituierten Oxy-dienen der Formel (IV), dadurch gekennzeichnet, daß man fluor-substituierte Carbonsäureester der Formel VI

$$R^1\text{-CO-OR}^2 \qquad \text{(VI)}$$

in welcher

$R^1$ und $R^2$ die oben angegebenen Bedeutungen haben,

mit 3-Methyl-but-2-enyl-triphenyl-phosphoniumbromid der Formel VII

$$(C_6H_5)_3\overset{\oplus}{P}\text{-}CH_2\text{-}CH{=}C{<}^{CH_3}_{CH_3} \qquad Br^{\ominus} \qquad \text{(VII)}$$

in Gegenwart einer starken Base und gegebenenfalls unter Verwendung von Verdünnungsmitteln umsetzt.

7. Insektizide und/oder akarizide Mittel, gekennzeichnet durch einen Gehalt an mindestens einem fluor-substituierten Oxyalkenyl-cyclopropan-carbonsäureester der Formel (I).

8. Verwendung von fluor-substituierten Oxyalkenyl-cyclopropancarbonsäureestern der Formel (I) zur Bekämpfung von Insekten und/oder Spinnentieren.

9. Verfahren zur Bekämpfung von Insekten und/oder Spinnentieren, dadurch gekennzeichnet, daß man fluor-substituierte Oxyalkenylcyclopropan-carbonsäureester der Formel (I) auf Insekten und/oder Spinnentiere und/oder ihren Lebensraum einwirken läßt.

10. Verfahren zur Herstellung insektizider und/oder akarizider Mittel, dadurch gekennzeichnet, daß man fluor-substituierte Oxyalkenylcyclopropan-carbonsäureester der Formel (I) mit Streckmitteln und/oder oberflächenaktiven Mitteln vermischt.

0020954

Europäisches Patentamt

**EUROPÄISCHER RECHERCHENBERICHT**

Nummer der Anmeldung

EP 80102449.8

| EINSCHLÄGIGE DOKUMENTE | | | KLASSIFIKATION DER ANMELDUNG (Int.Cl. 3) |
|---|---|---|---|
| Kategorie | Kennzeichnung des Dokuments mit Angabe, soweit erforderlich, der maßgeblichen Teile | betrifft Anspruch | |
| | DE - B - 2 109 010 (SUMITOMO)<br>+ Patentansprüche 1,2; Spalte 45, Zeile 28 bis Spalte 46, Zeile 9 +<br>-- | 1-3, 7-10 | C 07 C 69/743<br>C 07 C 43/17<br>C 07 C 61/35<br>A 01 N 53/00 |
| | DE - A - 2 230 862 (SUMITOMO)<br>+ Patentansprüche 1-14 +<br>-- | 1-3, 7-10 | |
| | DE - A1 - 2 634 663 (SUMITOMO)<br>+ Patentansprüche 1,2 +<br>-- | 4,5 | RECHERCHIERTE SACHGEBIETE (Int. Cl. 3) |
| | DE - A - 2 356 125 (ROUSSEL-UCLAF)<br>+ Patentansprüche 1,11 +<br>-- | 1,7-10 | C 07 C 69/00<br>C 07 C 43/00<br>A 01 N 53/00<br>C 07 C 61/00 |
| P | DE - A1 - 2 907 609 (MONTEDISON) (13-09-1979)<br>+ Patentansprüche 1-3,11-15,48, 52 +<br>& BE-A- 874 515 (28-08-1979)<br>& NL-A-7 901 460 (30-08-1979)<br>& GB-A-2 015 519 (12-09-1979)<br>-- | 1-3, 7-10 | |
| | DE - A1 - 2 650 534 (BAYER)<br>+ Patentansprüche 1-5; Seiten 8,9 +<br>-- | 1,2,4, 5,7-10 | KATEGORIE DER GENANNTEN DOKUMENTE<br>X: von besonderer Bedeutung<br>A: technologischer Hintergrund<br>O: nichtschriftliche Offenbarung<br>P: Zwischenliteratur<br>T: der Erfindung zugrunde liegende Theorien oder Grundsätze<br>E: kollidierende Anmeldung<br>D: in der Anmeldung angeführtes Dokument<br>L: aus andern Gründen angeführtes Dokument |
| | DE - B - 1 287 080 (DR. KARL THOMAE)<br>+ Patentanspruch +<br>-- | 5,6 | |
| X | Der vorliegende Recherchenbericht wurde für alle Patentansprüche erstellt. | | &: Mitglied der gleichen Patentfamilie, übereinstimmendes Dokument |

| Recherchenort | Abschlußdatum der Recherche | Prüfer |
|---|---|---|
| WIEN | 03-07-1980 | REIF |

Europäisches Patentamt

# EUROPÄISCHER RECHERCHENBERICHT

0020954

Nummer der Anmeldung

EP 80102449.8

| EINSCHLÄGIGE DOKUMENTE | | | KLASSIFIKATION DER ANMELDUNG (Int.Cl.X 3 |
|---|---|---|---|
| Kategorie | Kennzeichnung des Dokuments mit Angabe, soweit erforderlich, der maßgeblichen Teile | betrifft Anspruch | |
| P | DE - A1 - 2 851 428 (DAINIPPON) (13-06-1979)<br>+ Patentansprüche 1-17; Seiten 69-71 +<br>& FR-A-2 410 639 (29-06-1979)<br>& GB-A-2 010 262 (27-06-1979)<br>-- | 1-4, 7-10 | |
| D | DE - A - 2 326 077 (NATIONAL RESEARCH)<br>+ Patentansprüche 1,20,28,29 +<br>-- | 1-3, 7-10 | RECHERCHIERTE SACHGEBIETE (Int. ClX) 3 |
| D | DE - B - 1 926 433 (SUMITOMO)<br>+ Patentansprüche 1,2; Spalten 12,13 +<br>---- | 1-3, 7-10 | |